Europäisches Patentamt

European Patent Office

Office européen des brevets

(11) Publication number: **0 092 618**
**B1**

(12) # EUROPEAN PATENT SPECIFICATION

(45) Date of publication of patent specification: **22.04.87**

(51) Int. Cl.⁴: **A 61 M 16/04**

(21) Application number: **82302156.3**

(22) Date of filing: **27.04.82**

(54) **Esophageal-endotracheal airway.**

(43) Date of publication of application:
**02.11.83 Bulletin 83/44**

(45) Publication of the grant of the patent:
**22.04.87 Bulletin 87/17**

(84) Designated Contracting States:
**CH DE FR GB IT LI SE**

(56) References cited:
**CH-A- 541 332**
**DD-C- 68 597**
**DE-A-1 566 566**
**DE-A-2 120 164**
**FR-A-1 052 629**
**US-A-3 322 126**
**US-A-4 090 518**
**US-A-4 231 365**
**US-A-4 256 099**

(73) Proprietor: **Wallace, Patrick Albert**
**130 Charlotte Drive**
**Beaumont Texas 77705 (US)**

(73) Proprietor: **Bronson, Paul Allen**
**3002 Calder**
**Beaumont Texas 77701 (US)**

(72) Inventor: **Wallace, Patrick Albert**
**130 Charlotte Drive**
**Beaumont Texas 77705 (US)**
Inventor: **Bronson, Paul Allen**
**3002 Calder**
**Beaumont Texas 77701 (US)**

(74) Representative: **Rooney, Paul Blaise et al**
**D.Young & Co. 10 Staple Inn**
**London WC1V 7RD (GB)**

Courier Press, Leamington Spa, England.

## Description

An early airway for assisting in artificial respiration was made of an arcuate, open-ended tube which was adapted to be inserted into the trachea. This tube carried an external, inflatable, resilient sleeve near its distal end. The sleeve, when inflated, made a seal with the inner wall of the trachea.

Moving air through the trachea into and out of the respiratory orifices with this early airway required trained personnel, such as is normally found in hospital emergency rooms.

In U.S. Patents Nos. 3,683,908 and 3,841,319 is described an airway whose tube has a closed distal end. A plurality of openings are provided in the wall of the tube between its distal and proximal ends. The proximal end is the mouthpiece of the airway. The tube is shaped to become inserted into the esophagus which leads to the stomach. The tube carries near its distal end an inflatable sleeve. The sleeve, when inflated, can make a seal with the esophagus. Forced air passes from the inside to the outside of the tube through its openings and into the lungs of the patient. A mask is used to prevent the forced air from escaping through the patient's nose and mouth and to force the air to flow into the lungs.

But, if this close-ended tube of the airway were to be accidentally inserted into the trachea, as it may occasionally happen, the inflatable sleeve would seal off the trachea and the pumped air would flow into and inflate the stomach instead of the lungs. When depriving the lungs of air, the patient may die. Also, inflating the stomach may result in vomiting which alerts the attendant that the tubular member is in the trachea. The attendant will attempt to deflate the sleeve, remove the tube from the trachea, and insert it into the esophagus. But, even if this rescue operation is timely and successful, the vomiting from the patient's stomach can penetrate into the lungs and the patient may become seriously hurt or even die.

Also, when the tube is in the esophagus, the required mask for the nose and mouth may not provide an effective seal with the patient's face because face dimensions vary with every patient.

A problem can be also encountered even when the tube is inserted into the esophagus. If vomiting occurs during resuscitation, then high pressures become exerted from the stomach up through the esophagus. If the tube is in the esophagus with its sleeve inflated, vomitus cannot exit. Excessive pressure then becomes exerted at the junction zone between the stomach and esophagus. This high pressure can tear the esophagus away from the stomach which results in severe hemorrhaging.

Other airways are described in U.S. Patents Nos. 3,322,126 3,788,326, 3,905,361, 4,090,518, 4,231,365, 4,256,099, East German Patent No. 68,597 and DE—A—2120164 and CH—A—541332.

Of the latter Patents US—A—4090518 discloses an esophago-pharyngeal airway having an elongated stomach drain tube (15) and a shorter air tube (16) both passing in parallel side by side relationship through an oral flange (12) and through an attached proximal oral cuff or inflatable balloon (13). The elongated drain tube (15) also carries, near its distal end, an inflatable esophageal cuff or balloon (14) which in use when in the esophagus, engages the esophagus wall below the trachea mouth. The tube (16) in use in a patient terminates between the epiglottis and the base of the tongue with the balloon (13) sealing off the air passageways leading to the nose and mouth. The apparatus of US—A—4090518 is designed for ventilating the lungs, suctioning the stomach and defibrillating the heart with a low voltage. It is not suitable for blocking the esophagus whilst at the same time allowing a suction tube to be put in communication with the lungs and artificial respiration air to be pumped into and out of the lungs.

US—A—4231365 discloses an emergency resuscitation apparatus having an endotracheal tube and a laryngeal tube, which tubes are either in side-by-side relationship, or of the same length with a tracheal tube within the endotracheal tube, or with a shorter length laryngeal tube within a longer length endotracheal tube.

DD—C—68597 discloses a tracheal catheter only capable of operating in the trachea, having a pair of coaxial tubes with the distal end of the outer tube being secured and sealed to an inflatable bladder on the projecting end of the inner tube.

According to the present invention there is provided an esophagealendotracheal, flexible, tubular airway device including a shorter flexible tube (9) having an open-ended proximal end portion (9d) and an open-ended distal end portion (9a), which tube (9) is of a length such as to terminate, when inserted through the mouth of a patient, between the epiglottis and base of the tongue, a longer flexible tube (11) having an open-ended proximal end portion (11d) and an open-ended distal end portion (12), an expandable member (15a) disposed on the longer tube (11), for insertion through the mouth, which expandable member (15a) is operable for sealingly engaging, when projecting into the esophagus, the wall of the esophagus below the mouth of the trachea, and an expandable member (15b) disposed on the shorter tube (9) for simultaneously sealing off the air passageways leading to the nose and to the mouth of the patient, which device is characterised in that the longer tube (11) is dimensioned for insertion through the mouth into either the esophagus or the trachea of a patient, and the device is operable to provide separate passages for administering artificial respiration, stomach drainage and lung drainage simultaneously, the shorter flexible tube (9) has a larger diameter then the longer flexible tube (11), the smaller-diameter, longer flexible tube (11) is disposed inside said shorter tube (9) whereby the outer wall of said longer tube (11) and the inner wall of said shorter tube (9) define therebetween an annular or part annular passageway (9b) opening from the shorter tube distal end portion (9a) and said outer wall of the longer tube (11), such that a section tube (21) could be extended through said passageway (9b) and shorter tube distal end portion (9a) and out of said opening into said trachea or into said esophagus for evacuating fluid contents therefrom while administering artificial respiration to the lungs, with said opening being so dimensioned that even with a suction tube projecting through said opening into the trachea for evacuating fluid contents from the lungs artificial respiration air can still be passed through said opening down the trachea into the lungs, the expandable member (15a) disposed on said longer tube (11) is operable for sealingly engaging the wall of the trachea when projecting into the trachea and the expandable member (15b), disposed on said shorter tube (9) for simultaneously sealing off the air passageways leading to the nose and to the mouth of the patient, allowing with said member (15b) expanded and the longer tube (11) projecting into the esophagus, artificial respiration air to flow through said annular or part annular passageway (9b) into the patient's lungs.

On the outer wall near the distal end of each tubular member is disposed an inflatable sleeve. The inner tube extends through the wall of the outer tube near the proximal end of the outer tube. The proximal end of each tube is adapted to accept an air pump. Suction tubes can be inserted either through the inner tube or through the passageway formed between the walls of the tubes for pumping out the contents of the stomach or the lungs.

Ways of carrying out the invention are described with reference to the drawings which illustrate specific preferred embodiments and in which:

Fig. 1 is a perspective view of the double-tube airway of the present invention;

Fig. 2 is a transverse sectional view taken on line 2—2 of Fig. 1;

Fig. 3 is an enlarged, longitudinal, sectional view of a portion of the airway near the junction between both tubes;

Figs. 4 and 5 are fragmentary sectional views showing the air channels leading to the sleeves carried by the inner and outer tubes, respectively;

Fig. 6 shows the airway with its inner tube accidentally lodged in the trachea;

Fig. 7 shows the airway with its inner tube in the trachea for administering artificial resuscitation and carrying a suction tube for simultaneously suctioning the patient's stomach;

Fig. 8 shows the airway in its normal use while administering artificial resuscitation with its inner tube in the esophagus and both sleeves of the airway being inflated;

Fig. 9 is similar to Fig. 8 and in addition the airway carries a suction tube for suctioning the patient's lungs;

Fig. 10 is similar to Fig. 9 except that the stomach is now being suctioned;

Fig. 11 is similar to Fig. 8 except that vomitus is expelled to the atmosphere thereby preventing a dangerous pressure build up;

Fig. 12 is similar to Fig. 8 except that the proximal sleeve on the outer tube is deflated and a mask is used instead to cover the nose and mouth; and

Fig. 13 is similar to Fig. 2 but showing the airway's tubes as being eccentric.

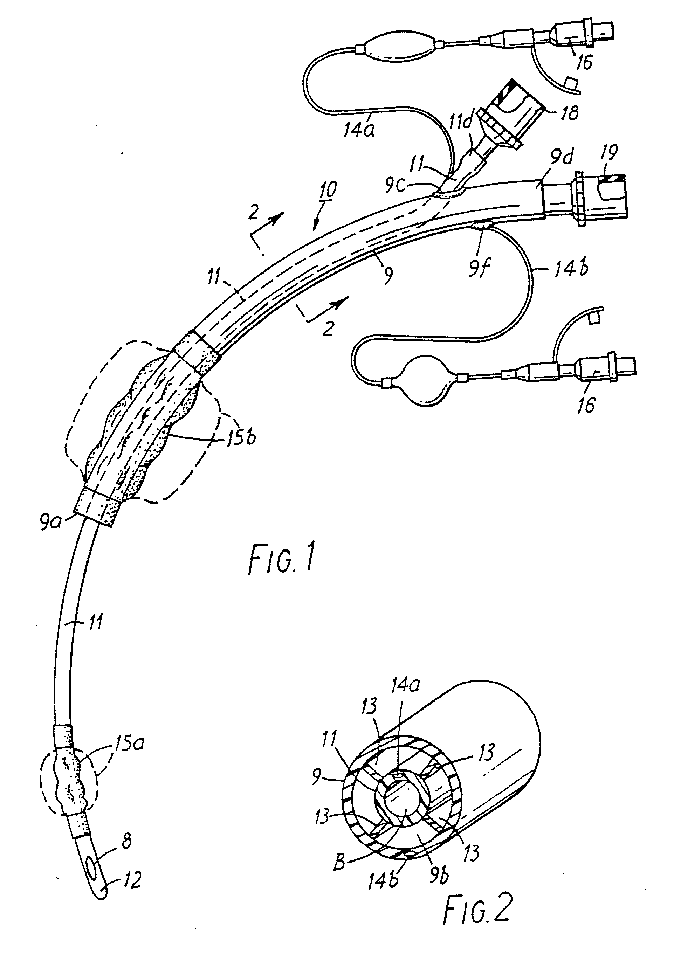

Figs. 1—5 show the esophageal-endotracheal airway of the present invention, generally designated as 10. It includes an inner, elongated, flexible tube 11 having a longitudinal bore B whose distal end 12 (Fig. 1) is open. An outer, shorter flexible tube 9 is coaxially mounted with the inner tube 11 (Fig. 2). The coaxiality between tubes 9, 11 is maintained by radial ribs 13. The distal end 9a of tube 9 is also open. Both tubes 9, 11 define an annular passageway 9b therebetween.

An expandable member, such as an inflatable sleeve 15a, is mounted externally near the distal end 12 on inner tube 11. Another inflatable sleeve 15b is mounted on tube 9 near the distal end 9a. Small-diameter hoses 14a and 14b are adapted to carry pressurized air for inflating sleeves 15a and 15b, respectively. Each hose 14a or 14b is coupled to a check valve 16 (Fig. 1). When sleeves 15a, 15b expand, as represented by the dotted lines, they form effective seals with the adjacent surrounding wall of the body channel. As will be subsequently described, the airway 10 can seal off the nose and mouth without the assistance of a facial mask.

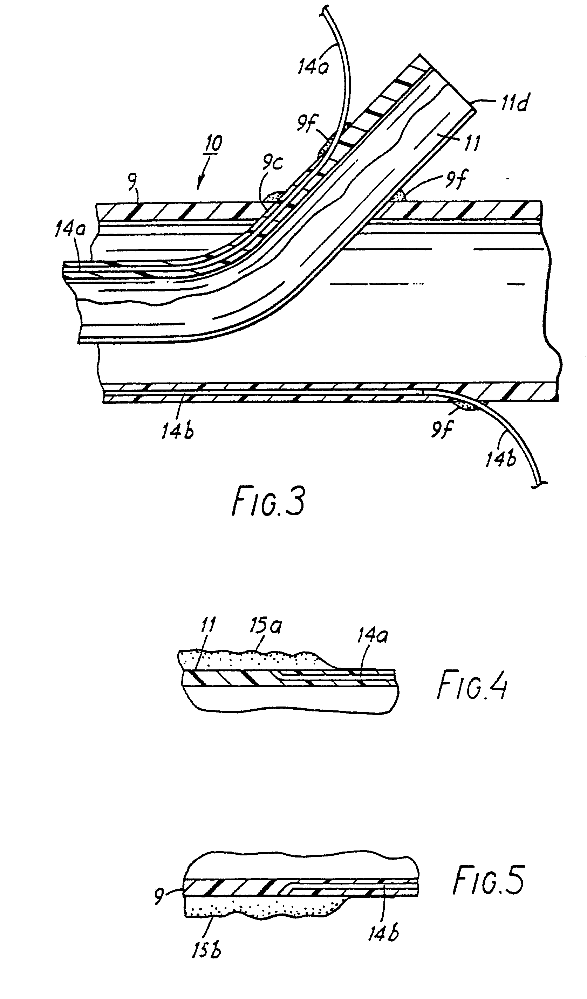

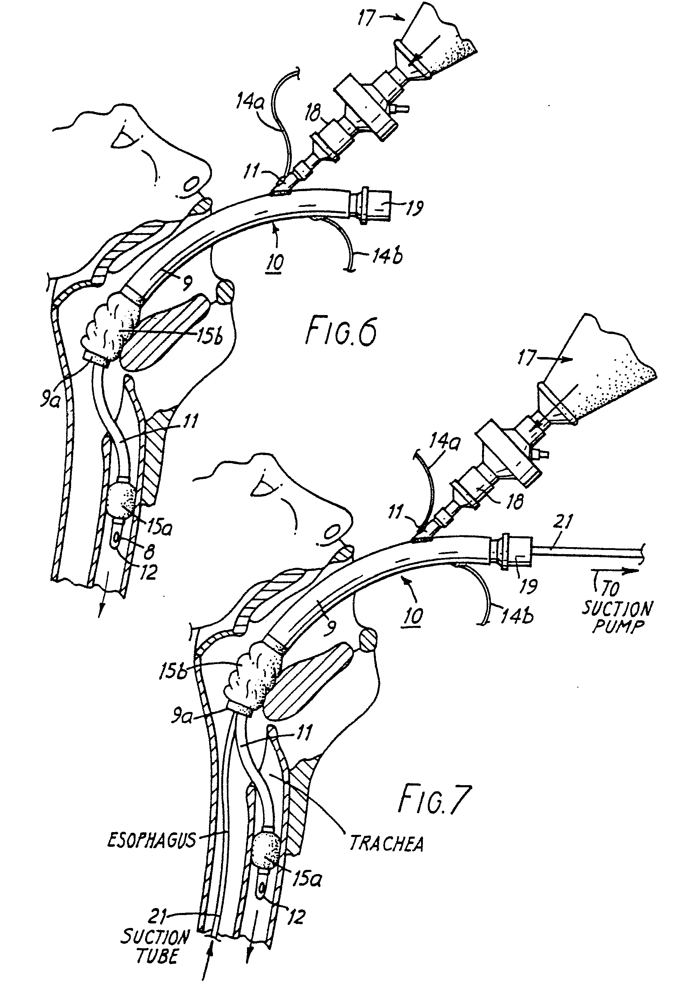

The inner tube 11 extends through an opening 9c (Fig. 3) in the wall of the outer tube 9 near its proximal end 9d. Tubes 9 and 11 form a fluid-tight joint 9f therebetween. The proximal ends 9d and 11d of the tubes 9 and 11 are coupled to adapters 19 and 18, respectively. Each adapter can receive the outlet of a conventional air pump 17 (Figs. 6—12). Tube 11 has a hole 8 therethrough in a direction perpendicular to its longitudinal axis in order to make its distal end 12 more flexible.

In use, when inner tube 11 by accident becomes inserted into the trachea (Fig. 6), the attendant need only inflate sleeve 15a. Resuscitation with pump 17 can be produced through bore B (Fig. 2) of tube 11.

Fig. 7 shows that while resuscitation is being administered to the patient, the contents of the stomach can be emptied by inserting into the esophagus, through the annular passageway 9b, a suction tube 21 suitably coupled to a conventional suction pump (not shown).

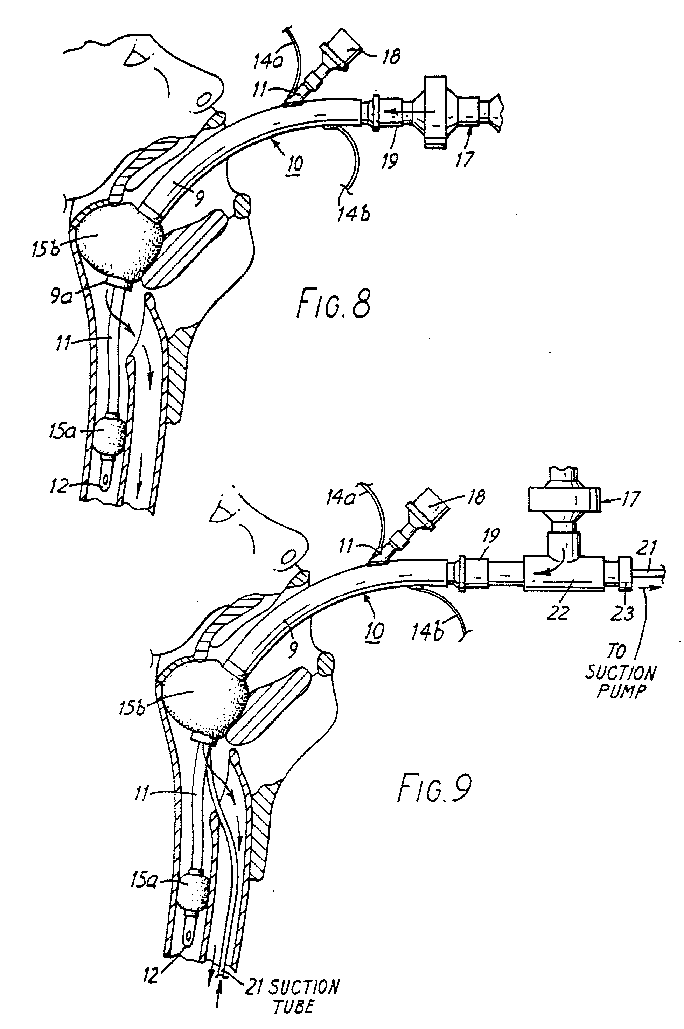

Fig. 8 shows the normal position of airway 10 for administering artificial resuscitation. Tube 11 is inserted into the esophagus. Both sleeves 15a, 15b are inflated. Air is pumped by pump 17 into tube 9. This forced air passes from the distal end 9a of outer tube 9 through the trachea and into the lungs.

Fig. 9 is similar to Fig. 8 except that a suction tube 21 is now inserted through a T-coupling member 22 into tube 9 and exists from its distal end 9a into the trachea of the patient. The T-coupling 22 has a packing gland 23. In this manner, artificial resuscitation takes place simultaneously with the suctioning of the lungs.

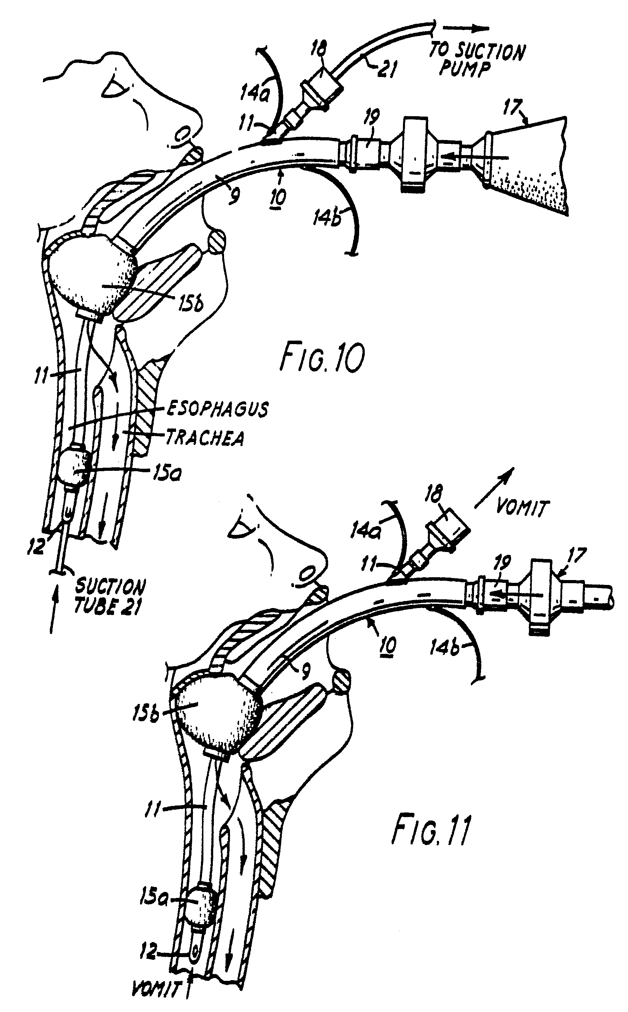

As an immediate advantage of the airway 10, even when the inner tube 11 by accident becomes inserted into the trachea (Fig. 6), the respiratory function of the airway can still be carried out, and in addition, the contents of the lungs and the contents of the stomach (Fig. 7) can be suctioned out. Also, during normal use of the airway (Fig. 8), artificial respiration can be maintained, while the contents of the lungs (Fig. 9) or the contents of the stomach (Fig. 10) are being suctioned out.

Fig. 11 is similar to Fig. 8 except that regurgitation has taken place already with vomitus pouring out from the proximal end of the inner tube 11. But this vomitus neither produces contamination of the posterior pharynx nor increases the pressure at the junction between the stomach and the esophagus.

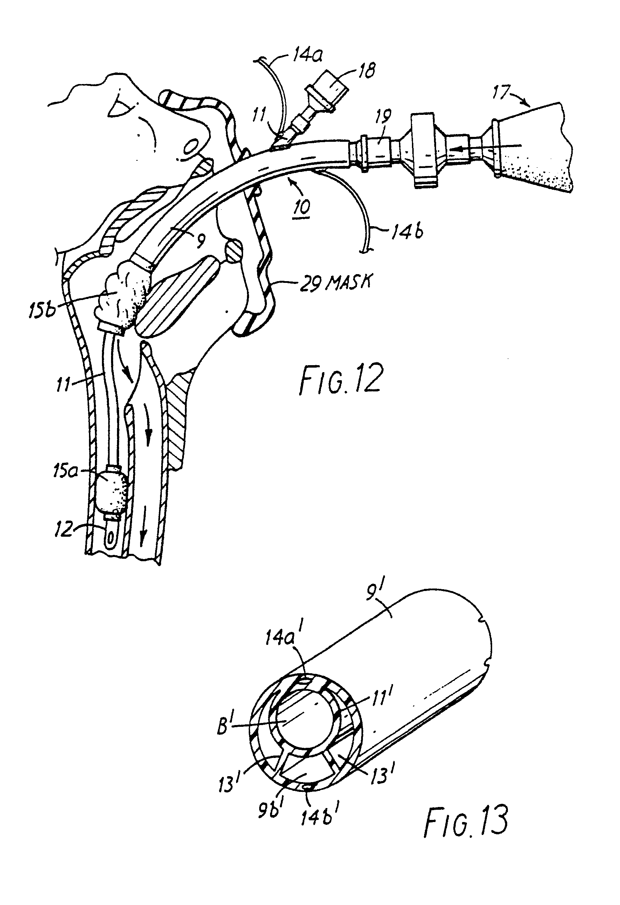

Instead of inflating sleeve 15b as in Fig. 8, a mask 29 (Fig. 12) can be employed. No excessive pressure will then develop at the union between the stomach and the esophagus during vomiting, because bore B (Fig. 2) of tube 11 remains open to the atmosphere while tube 11 is being inserted into the esophagus.

When sleeve 15a is inflated it creates a seal against the esophageal wall and vomitus cannot contaminate the posterior pharynx.

In Fig. 13 is shown a modification of the airway structure wherein the same numerals followed by a prime (') are used to designate the same or similar members. Each thusly designated member serves a function as previously described. It will be noted that tubes 9' and 11' are not concentrically mounted. In fact, the tubes can have their walls in touching relation as shown.

## Claims

1. An esophageal-endotracheal, flexible, tubular airway device including a shorter flexible tube (9) having an open-ended proximal end portion (9d) and an open-ended distal end portion (9a), which tube (9) is of a length such as to terminate, when inserted through the mouth of a patient, between the epiglottis and base of the tongue, a longer flexible tube (11) having an open-ended proximal end portion (11d) and an open-ended distal end portion (12), an expandable member (15a) disposed on the longer tube (11), for insertion through the mouth, which expandable member (15a) is operable for sealingly engaging, when projecting into the esophagus, the wall of the esophagus below the mouth of the trachea, and an expandable member (15b) disposed on the shorter tube (9) for simultaneously sealing off the air passageways leading to the nose and to the mouth of the patient, which device is characterised in that the longer tube (11) is dimensioned for insertion through the mouth into either the esophagus or the trachea of a patient, and the device is operable to provide separate passages for administering artificial respiration, stomach drainage and long drainage simultaneously, the shorter flexible tube (9) has a larger diameter than the longer flexible tube (11), the smaller-diameter, longer flexible tube (11) is disposed inside said shorter tube (9) whereby the outer wall of said longer tube (11) and the inner wall of said shorter tube (9) define therebetween an annular or part annular passageway (9b) opening from the shorter tube distal end portion (9a) and said outer wall of the longer tube (11), such that a section tube (21) could be extended through said passageway (9b) and shorter tube distal end portion (9a) and out of said opening into said trachea or into said esophagus for evacuating fluid contents therefrom while administering artificial respiration to the lungs, with said opening being so dimensioned that even with a suction tube projecting through said opening into the trachea for evacuating fluid contents from the lungs artificial respiration air can still be passed through said opening down the trachea into the lungs, the expandable member (15a) disposed on said longer tube (11) is operable for sealingly engaging the wall of the trachea when projecting into the trachea and the expandable member (15b), disposed on said shorter tube (9) for simultaneously sealing off the air passageways leading to the nose and to the mouth of the patient, allowing with said member (15b) expanded and the longer tube (11) projecting into the esophagus, artificial respiration air to flow through said annular or part annular passageway (9b) into the patient's lungs.

2. The airway device of claim 1, wherein the proximal end portion (11d) of the tube (11) opens through the wall of the tube (9) adjacent the proximal end portion (9d), thereof, and including a suction tube (21) removably extending through the longer tube (11), which suction tube (21) is operably positionable for suctioning the stomach when said longer tube (11) is in the esophagus while administering artificial respiration to the lungs through said annular or part annular passageway and said opening thereof, the suction tube (21) entering the tube (11) through the proximal end portion (11d) thereof and exiting from the tube (11) through the distal end portion (12) thereof.

3. The airway device of claim 1 or claim 2, including a suction tube (21) removably extending through said annular or part annular passageway (9b) from said proximal end portion (9d) and exiting through said opening, which suction tube (21) is operably positionable for suctioning the stomach when said longer tube (11) is in the trachea while administering artificial respiration to the lungs through said longer tube (11), or is operably positionable for suctioning the lungs when said longer tube (11) is in the esophagus while administering artificial respiration to the lungs through the annular or part annular passageway (9b).

## Patentansprüche

1. Vorrichtung mit einem ösophagischen-endotrachealen flexiblen, rohrförmigen Luftkanal, mit einem kürzeren flexiblen Schlauch (9) mit einem offenendigen, nächstgelegenen Endteil (9d) und einem offenendigen distalen Endteil (9a), wobei der Schlauch (9) eine solche Länge hat, daß er, wenn er durch den Mund eines Patienten eingeführt ist, zwischen der Epiglottis und der Basis der Zunge endet, mit einem längeren flexiblen Schlauch (11) mit einem offenendigen nächstgelegenen Endteil (11d) und einem offenendigen distalen Endteil (12), wobei ein expandierbares Teil (15a) auf dem längeren Schlauch (11) angeordnet ist zum Einführen durch den Mund, das expandierbare Teil (15a) beim Hineinragen in den Esophagus für den dichtenden Eingriff mit der Wand des Esophagus unter dem Mund der Trachea betätigbar ist und ein expandierbares Teil (15b) auf dem kürzeren Schlauch (9) angeordnet ist zum gleichzeitigen Abdichten der Luftdurchgänge, welche zu der Nase und zum Mund des Patienten führen, dadurch gekennzeichnet, daß der längere Schlauch (11) für das Einführen durch den Mund entweder in den Esophagus oder in die Trachea eines Patienten bemessen ist und die Vorrichtung betreibbar ist, um separate Durchgänge vorzusehen, um der künstlichen Beatmung zu dienen, Magendrainage und Lungendrainage gleichzeitig vorzusehen, der kürzere flexible Schlauch (9) einen größeren Durchmesser hat als der längere flexible Schlauch (11), der längere flexible Schlauch (11) mit dem kleineren Durchmesser innerhalb des kürzeren Schlauches (9) angeordnet ist, wodurch die Außenwand des längeren Schlauches (11) und die Innenwand des kürzeren Schlauches (9) dazwischen einen ringförmigen oder teilringförmigen Durchgang (9b) bilden, der sich von dem distalen Endteil (9a) des kürzeren Schlauches und der Außenwand des längeren Schlauches (11) öffnet, derart, daß ein Saugschlauch (21) durch diesen Durchgang (9b) und den distalen Endteil (9a) des kürzeren Schlauches und aus der Öffnung in die Trachea hinein oder in den Esophagus hinein geführt werden könnte, um Fluidinhalt aus diesen zu entleeren, während die künstliche Bestmung zu den Lungen gewährleistet wird, wobei die Öffnung so bemessen ist, daß sogar bei einem Saugschlauch, welcher durch die Öffnung in die Trachea hineinragt zum Entleeren des Fluidinhaltes aus den Lungen, die Luft der künstlichen Beatmung noch durch die Öffnung die Trachea hinab in die Lungen hinein-geführt werden kann, das expandierbare Teil (15a), welches auf dem längeren Schlauch (11) angeordnet ist, für den abdichtenden Eingriff der Wand der Trachea betätigbar ist, wenn er in die Trachea ragt, und das expandierbare Teil (15b), welches auf dem kürzeren Schlauch (9) angeordnet ist, für das gleichzeitige Abdichten der Luftdurchgänge, welche zur Nase und zum Mund des Patienten führen, und daß bei expandiertem Teil (15b) und Hineinragen des längeren Schlauches (11) in den Esophagus die Luft der künstlichen Beatmung durch den ringförmigen oder teilringförmigen Durchgang (9b) in die Lungen des Patienten hineinströmen kann.

2. Luftkanalvorrichtung nach Anspruch 1, wobei das nächstgelegene Endteil (11d) des Schlauches (11) sich durch die Wand des Schlauches (9) neben dem nächstgelegenen Endteil (9d) desselben öffnet und einen Saugschlauch (21) aufweist, der sich abnehmbar durch den längeren Schlauch (11) erstreckt, wobei der Saugschlauch (21) zum Aussaugen des Magens betrieblich positionierbar ist, wenn der längere Schlauch (11) sich im Esophagus befindet, während die künstliche Beatmung zu den Lungen durch den ringförmigen oder teilringförmigen Durchgang und seine Öffnung gewährleistet ist, der Saugschlauch (21) in den Schlauch (11) durch das nächstgelegene Endteil (11d) desselben eintritt und aus dem Schlauch (11) durch das distale Endteil (12) desselben austritt.

3. Luftkanalvorrichtung nach Anspruch 1 oder Anspruch 2, mit einem Saugschlauch (21), der sich entfernbar durch den ringförmigen oder teilringförmigen Durchgang (9b) von dem nächstgelegenen Endteil (9d) erstreckt und durch die Öffnung austritt, wobei der Saugschlauch (21) für das Aussaugen des Magens betrieblich einstellbar ist, wenn sich der längere Schlauch (11) in der Trachea befindet, während die künstliche Beatmung zu den Lungen durch den längeren Schlauch (11) durchgeführt wird, oder für das Absaugen der Lungen betrieblich einstellbar ist, wenn sich der längere Schlauch (11) in dem Esophagus befindet, während die künstliche Beatmung zu den Lungen durch den ringförmigen oder teilringförmigen Durchgang (9b) vorgesehen wird.

## Revendications

1. Dispositif conduit de respiration oesophagie-nendotrachéal tubulaire, flexible comprenant un tube flexible plus court (9) ayant une partie d'extrémité proximale (9d) à extrémité ouverte et une partie d'extrémité distale (9a) à extrémité ouverte, lequel tube (9) est d'une longueur telle qu'il se termine, une fois introduit par la bouche d'un patient, entre l'épiglotte et la base de la langue, un tube flexible plus long (11) ayant une partie d'extrémité proximale (11d) à extrémité ouverte et une partie d'extrémité distale (12) à extrémité ouverte, un élément dilatable (15a) placé sur ledit tube plus long (11), pour introduction par la bouche, lequel élement dilatable (15a) est agencé pour engager de façon étanche, une fois introduit dans l'oesophage, la paroi de l'oesophage en dessous de l'embouchure de la trachée, et un élément dilatable (15b) placé sur ledit tube court (9) pour fermer simultanément les passages d'air conduisant au nez et à la bouche du patient, lequel dispositif est caractérisé en ce que le tube plus long (11) est dimensionné pour introduction par la bouche soit dans l'oesophage, soit dans la trachée du patient, et le dispositif est adapté à fournir des passages séparés pour réaliser simul-

**0 092 618**  10

tanément une respiration artificielle, un drainage de l'estomac et un drainage des poumons, ledit tube flexible plus court (9) a un diamètre supérieur à celui dudit tube flexible plus long (11), ledit tube flexible plus long (11) de plus faible diamètre est disposé au dedans dudit tube plus court (9), si bien que la paroi externe dudit tube plus long (11) et la paroi interne dudit tube plus court (9) définissent entre elles un passage annulaire ou en partie annulaire (9b) s'ouvrant entre la partie d'extrémité distale (9a) du tube plus court et ladite paroi externe du tube plus long (11), de sorte qu'un tube de succion (21) puisse être passé, au travers dudit passage (9b) et de la partie d'extrémité distale (9a) du tube plus court et au-delà de ladite ouverture dans ladite trachée ou dans ledit oesophage pour en évacuer des contenus liquides alors que les poumons sont placés sous respiration artificielle, ladite ouverture étant dimensionnée d'une façon telle que, même avec un tube de succion faisant saillie à travers ladite ouverture dans la trachée pour évacuer des contenus liquides des poumons, l'air de respiration artificielle peut encore être amené par ladite ouverture en descendant le long de la trachée dans les poumons, ledit élément dilatable (15a) placé sur ledit tube plus long (11) étant agencé pour engager de façon étanche la paroi de la trachée lorsqu'il est avancé dans ladite trachée et l'élément dilatable (15b), placé sur ledit tube plus court (9) pour fermer simultanément les passages d'air conduisant au nez et à la bouche du patient, permettant avec ledit élément (15b) dilaté et le tube plus long (11) pénétrant dans l'oesophage, à l'air de respiration artificielle de circular à travers ledit passage (9b) annulaire ou en partie annulaire vers les poumons du patient.

2. Dispositif conduit de respiration selon la revendication 1, dans lequel ladite partie d'extrémité proximale (11d) tu tube (11) débouche à travers la paroi du tube (9) au voisinage de la partie d'extrémité terminale (9b) de celui-ci, et comprenant un tube de succion (21) s'étendant de façon amovible à travers ledit tube plus long (11), lequel tube de succion (21) est adapté à être placé en position de succion dans l'estomac lorsque ledit tube plus long (11) est dans l'oesophage alors que les poumons sont placés sous respiration artificielle à travers ledit passage annulaire ou en partie annulaire et sa dite ouverture, le tube de succion (21) pénétrant dans le tube (11) par sa partie d'extrémité proximale (11d) et sortant du tube (11) par la partie d'extrémité distale (12) de ce dernier.

3. Dispositif conduit de respiration selon la revendication 1 ou 2, comprenant un tube de succion (21) s'étendant de façon amovible à travers ledit passage (9b) annulaire ou en partie annulaire à partir de ladite partie d'extrémité proximale (9d) et sortant par ladite ouverture, lequel tube de succion (21) est adapté à être positionné pour succion dans l'estomac, lorsque ledit tube plus long (11) est dans la trachée en administrant aux poumons de la respiration artificielle à travers ledit tube plus long (11), ou pouvant être positionné pour succion dans les poumons lorsque ledit tube plus long (11) est dans l'oesophage en administrant aux poumons de la respiration artificielle à travers ledit passage annulaire ou partiellement annulaire (9b).

FIG. 1

FIG. 2

FIG.3

FIG.4

FIG.5

*17*

*14a*  *18*

*11*

*19*

*10*

*9*

*15b*

*FIG.6*

*9a*

*11*

*15a*

*8*

*12*

*17*

*14a*

*11*  *18*

*21*

*19*

*TO SUCTION PUMP*

*10*

*9*

*15b*

*9a*

*11*

*FIG.7*

*15b*

*ESOPHAGUS*

*TRACHEA*

*15a*

*21*

*12*

*SUCTION TUBE*

FIG.8

FIG.9

TO
SUCTION
PUMP

21 SUCTION
TUBE

4

FIG. 10

FIG. 11

*14a*

*18*

*11*

*17*

*19*

*10*

*9*

*14b*

*29 MASK*

*15b*

*11*

*FIG.12*

*15a*

*12*

*9¹*

*14a¹*

*11¹*

*B¹*

*13¹*

*13¹*

*9b¹*

*14b¹*

*FIG.13*